Europäisches Patentamt

European Patent Office

Office européen des brevets

(19)

(11) **EP 0 750 941 B1**

(12) **FASCICULE DE BREVET EUROPEEN**

(45) Date de publication et mention de la délivrance du brevet:
**12.11.2003 Bulletin 2003/46**

(51) Int Cl.[7]: **B01J 27/128**, C07C 5/27

(21) Numéro de dépôt: **96401327.0**

(22) Date de dépôt: **19.06.1996**

(54) **Catalyseur à base d'alumine chlorée, et son utilisation en isomérisation des normales paraffines C4-C6**

Chloriertes Alumineiumoxid enthaltender Katalysator und seine Anwendung zur Isomerisierung von C4-C6 normalen Paraffinen

Chlorinated alumina based catalyst, and its use in the isomerization of normal C4-C6 paraffins

(84) Etats contractants désignés:
**DE ES GB IT NL**

(30) Priorité: **28.06.1995 FR 9507887**

(43) Date de publication de la demande:
**02.01.1997 Bulletin 1997/01**

(73) Titulaire: **Institut Français du Pétrole**
**92502 Rueil-Malmaison (FR)**

(72) Inventeurs:
• **Cauffriez, Hervé**
**78380 Bougival (FR)**

• **Travers, Christine**
**92500 Rueil Malmaison (FR)**

(56) Documents cités:
WO-A-91/17825      US-A- 3 963 643
US-A- 4 003 849      US-A- 4 039 604

Remarques:
Le dossier contient des informations techniques présentées postérieurement au dépôt de la demande et ne figurant pas dans le présent fascicule.

**Description**

La présente invention concerne un catalyseur à base d'alumine chlorée et sa mise en oeuvre dans un procédé d'isomérisation de normales paraffines $C_4$-$C_6$.

L'isomérisation des normales paraffines comportant entre 4 et 6 atomes de carbone par molécule revêt actuellement une importance considérable dans l'industrie pétrolière, principalement à cause de la suppression des alkyls de plomb dans les essences.

L'isomérisation du n-butane permet de produire de l'isobutane respectivement pour l'alkylation aliphatique des oléfines et pour la synthèse du MTBE (méthyl tertio butyl éther) via la deshydrogénation de l'isobutane, permettant respectivement de produire un alkylat d'indice d'octane élevé et de fournir du MTBE, composés incorporables aux fractions essences.

L'isomérisation des normales paraffines $C_5$-$C_6$ permet de transformer des paraffines de faible indice d'octane en isoparaffines d'indice d'octane élevé.

Trois types de catalyseurs sont traditionnellement utilisés pour réaliser la réaction d'isomérisation des normales paraffines comportant entre 4 et 6 atomes de carbone par molécule, de préférence entre 5 et 6 atomes de carbone par molécule:

- les catalyseurs de type Friedel et Crafts, tel que le chlorure d'aluminium, qui sont utilisés à des basses températures (environ 80 à 130°C);

- les catalyseurs comprenant au moins un métal du groupe VIII sur support à base d'alumine halogénée, de préférence chlorée, qui sont utilisés à des températures moyennes (environ 150°C),

- les catalyseurs zéolithiques comprenant au moins un métal du groupe VIII déposé sur une zéolithe, qui sont utilisés à des températures élevées (250°C et plus); lesdits catalyseurs conduisent à des gains d'octane plus faibles dans les produits obtenus que les deux types de catalyseurs précédemment décrits mais présentent l'avantage d'être plus faciles à mettre en oeuvre et d'être plus résistants aux poisons. Néanmoins, ils ne peuvent pas être employés pour l'isomérisation du n-butane car ils développent en plus une plus faible acidité que les deux types de catalyseurs précédemment décrits.

De nombreux brevets ont pour objet des catalyseurs monométalliques à base de platine déposé sur une alumine halogénée, et leur utilisation dans des procédés d'isomérisation des normales paraffines. On peut citer le brevet US-A-3 963 643, qui impose un traitement par un composé de type Friedel et Crafts suivi par un traitement avec un composé chloré comportant au moins deux atomes de chlore.

Plus récemment le brevet US-A-5 166 121 revendique un catalyseur comprenant de l'alumine gamma mise en forme sous forme de billes et comportant entre 0,1 et 3,5 % d'halogène sur le support. La teneur en halogène de préférence en chlore déposé sur le support est extrêmement faible.

L'invention concerne un catalyseur contenant au moins du chlore, au moins un métal du groupe VIII et un support mis en forme comprenant de l'alumine gamma et éventuellement de l'alumine êta, le catalyseur étant caractérisé en ce que la plus petite dimension moyenne dudit support est comprise entre 0,8 et 2 mm, de préférence entre 1 et 1,8 mm, et en ce que sa teneur en chlore est comprise entre 4,5 et 15%, de préférence entre 5 et 12%, en poids.

Le support du catalyseur selon l'invention est à base d'alumine, c'est-à-dire qu'il comprend essentiellement de l'alumine. Le support d'alumine est de l'alumine gamma à laquelle s'ajoute éventuellement de l'alumine êta. Lorsque l'on ajoute de l'alumine êta à l'alumine gamma, l'alumine du support comprend généralement entre 50 et 100% (borne exclue), de préférence entre 80 et 100% (borne exclue) (% poids) d'alumine êta, le complément étant de l'alumine gamma.

La plus petite dimension moyenne du support du catalyseur selon l'invention est comprise entre 0,8 et 2mm, de préférence entre 1 et 1,8 mm. De préférence, ledit support est formé essentiellement de billes de diamètre moyen compris entre 0,8 et 2 mm, de préférence entre 1 et 1,8 mm, ou bien ledit support est formé essentiellement d'extrudés dont la plus petite dimension est comprise entre 0,8 et 2 mm, de préférence entre 1 et 1,8 mm, c'est-à-dire que les extrudés ont été mis en forme à partir de toute technique d'extrusion connue de l'homme du métier, comme par exemple une filière de diamètre compris entre 0,8 et 2 mm, de préférence entre 1 et 1,8 mm.

L'alumine gamma présente dans le support du catalyseur selon l'invention possède une surface spécifique généralement comprise entre 150 et 300 m²/g et de préférence entre 180 et 250 m²/g , et un volume poreux total généralement compris entre 0,4 et 0,8 cm³/g et de manière préférée entre 0,45 et 0,7 cm³/g.

L'alumine êta éventuellement présente dans le support du catalyseur selon l'invention possède une surface spécifique généralement comprise entre 400 et 600 m²/g et de préférence entre 420 et 550 m²/g et un volume poreux total généralement compris entre 0,3 et 0,5 cm³/g et de manière préférée entre 0,35 et 0,45 cm³/g.

Le métal du groupe VIII est choisi dans le groupe formé par le fer, le cobalt, le nickel, le ruthénium, le rhodium, le palladium, l'osmium, l'iridium et le platine, de préférence choisi dans le groupe formé par le platine, le palladium et le nickel. Dans le cas préféré où ledit métal est le platine ou le palladium, la teneur en poids est comprise entre 0,05 et 1 % et de manière préférée entre 0,1 et 0,6 %. Dans le cas préféré où ledit métal est le nickel, la teneur pondérale est comprise entre 0,1 et 10 % et de manière préférée entre 0,2 et 5 %.

Le catalyseur selon l'invention comprend au moins du chlore, à une teneur comprise entre 4,5 et 15% poids de préférence comprise entre 5 et 12 % poids.

La préparation du catalyseur selon l'invention s'effectue généralement par mise en forme du support, puis par dépôt sur ledit support mis en forme d'au moins un métal du groupe VIII, et enfin par chloration, après une étape éventuelle préférée d'activation sous hydrogène. Chaque étape du procédé de préparation du support selon l'invention est explicitée ci-après.

Dans le cas où de l'alumine êta est présente dans le support du catalyseur selon l'invention, les deux types d'alumine sont de préférence mélangées et mises en forme ensemble, selon toute technique connue de l'homme du métier, par exemple par extrusion au travers d'une filière, par pastillage ou par dragéification. Mais il est aussi possible de mettre les deux types d'alumine en forme séparément, puis de procéder au mélange des deux alumines mises en forme. Dans tous les cas, la plus petite dimension de la forme géométrique décrite par le support après mise en forme est comprise entre 0,8 et 2 mm, de préférence entre 1 et 1,8 mm, ce qui permet d'obtenir, lors de l'étape de chloration du support, une teneur en chlore suffisante pour une durée de chloration réduite.

Au moins un métal hydrogénant du groupe VIII, de préférence choisi dans le groupe formé par le platine, le palladium, et le nickel, est ensuite déposé sur ce support par toute technique connue de l'homme du métier, par exemple par échange anionique sous forme d'acide hexachloroplatinique dans le cas du platine ou sous forme de chlorure dans le cas du palladium.

Une fois le dépôt du (des) métal (métaux) effectué, le catalyseur peut éventuellement subir un traitement d'activation sous air à haute température, par exemple à une température comprise entre 300 et 700°C, puis un traitement sous hydrogène afin d'obtenir une phase métallique active. La procédure de ce traitement sous hydrogène comprend par exemple une montée lente de la température sous courant d'hydrogène jusqu'à la température maximale de réduction comprise généralement entre environ 300 et 700°C, de préférence entre environ 340 et 680°C, suivie d'un maintien de cette température, généralement pendant 1 à 6 heure(s), de préférence pendant 1,5 à 4,5 heures.

La chloration, de l'alumine est effectuée en dehors de ou directement dans l'unité de réaction où l'on utilise le catalyseur, de préférence une unité d'isomérisation, par le tétrachlorure de carbone ou le chloroforme, ainsi qu'il est exemplifié ci-après.

Le traitement de chloration, peut être effectué directement dans l'unité d'utilisation du catalyseur selon l'invention, avant injection de la charge, ou hors site.

L'utilisation d'un support caractérisé en ce que sa plus petite dimension moyenne est comprise entre 0,8 et 2 mm, de préférence entre 1 et 1,8 mm, et en ce que sa teneur en chlore, est comprise entre 4,5 et 15%, de préférence entre 5 et 12%, en poids, l'agent chlorant étant le tétrachlorure de carbone ou le chloroforme permet avantageusement d'obtenir une chloration, rapide et uniforme. L'utilisation d'un support dont la plus petite dimension moyenne est supérieure à 2 mm ne permet pas une halogénation rapide et totale du support; la teneur en halogène (pourcentage poids) du support restant alors inférieure à 4,5 % même après un temps d'halogénation très long, ce qui ne permet pas d'obtenir l'activité et la sélectivité maximale pour ce type de catalyseur.

Dans le procédé de préparation du catalyseur selon l'invention, il est aussi possible de procéder au traitement de chloration préalablement au traitement d'activation puis de réduction sous hydrogène. Dans ce cas, le traitement de réduction sous hydrogène peut avoir lieu en dehors de l'unité (ex situ"), ce qui implique de prendre des précautions particulières pour le transport dudit catalyseur jusqu'à ladite unité, ou bien ledit traitement peut avoir lieu au sein de l'unité ("in-situ") juste avant l'utilisation dudit catalyseur.

Le catalyseur selon l'invention est utilisé dans un procédé conventionnel d'isomérisation d'une charge comprenant en majeure partie des normales paraffines comportant de 4 à 6 atomes de carbone par molécule, de préférence de 5 à 6 atomes de carbone par molécule, dont on indique ci-après les conditions opératoires usuelles.

L'isomérisation a lieu dans au moins un réacteur. La température est comprise entre 100 et 300°C, de préférence entre 120 et 280°C, et la pression partielle d'hydrogène est comprise entre la pression atmosphérique et 7 MPa, de préférence entre 0,5 et 5 MPa. La vitesse spatiale est comprise entre 0,2 et 10 litres, de préférence entre 0,5 et 5 litres d'hydrocarbures liquides par litre de catalyseur et par heure. Le rapport molaire hydrogène/charge à l'entrée du réacteur est tel que le rapport molaire hydrogène/charge dans l'effluent sortant du réacteur est supérieur à 0,06, de préférence compris entre 0,06 et 10.

Les exemples qui suivent précisent l'invention sans en limiter la portée.

EXEMPLE 1 Catalyseur A (conforme à l'invention)

L'alumine gamma est mise en forme par extrusion au travers d'une filière de diamètre 1,2 mm. Après calcination sous air préalable, on dépose sur ladite alumine mise en forme 0,3 % de platine par échange ionique avec de l'acide hexachloroplatinique en présence d'HCl comme agent compétiteur. Le solide ainsi préparé est calciné sous air puis réduit sous hydrogène à 600°C.
On procède ensuite à la chloration du solide obtenu, à une température de 250°C, par injection de tétrachlorure de carbone.

EXEMPLE 2 Catalyseur B (non conforme à l'invention)

L'alumine gamma est mise en forme par extrusion au travers d'une filière de diamètre 1,2 mm. Après calcination sous air préalable, on dépose sur ladite alumine mise en forme 0,3 % de platine par échange ionique avec de l'acide hexachloroplatinique en présence d'HCl comme agent compétiteur. Le solide ainsi préparé est calciné sous air puis réduit sous hydrogène à 600°C.
On procède ensuite à la chloration du solide obtenu, à une température de 250°C, par injection de chlorure d'hydrogène.
L'utilisation de chlorure d'hydrogène rend non conforme à l'invention le catalyseur ainsi préparé.

EXEMPLE 3 Catalyseur C (non conforme à l'invention)

L'alumine gamma est mise en forme par extrusion au travers d'une filière de diamètre 2,4mm. Après calcination sous air préalable, on dépose sur ladite alumine mise en forme 0,3 % de platine par échange ionique avec de l'acide hexachloroplatinique en présence d'HCl comme agent compétiteur. Le solide ainsi préparé est calciné sous air puis réduit sous hydrogène à 600°C.
On procède ensuite à la chloration du solide obtenu, à une température de 250°C, par Injection de tétrachlorure de carbone.
L'utilisation d'une filière de diamètre 2,4 mm rend non conforme à l'invention le catalyseur ainsi préparé.

EXEMPLE 4 Catalyseur D (non conforme à l'invention)

L'alumine gamma est mise en forme par extrusion au travers d'une filière de diamètre 2,4 mm. Après calcination sous air préalable, on dépose sur ladite alumine mise en forme 0,3 % de platine par échange ionique avec de l'acide hexachloroplatinique en présence d'HCl comme agent compétiteur. Le solide ainsi préparé est calciné sous air puis réduit sous hydrogène à 600°C.
On procède ensuite à la chloration du solide obtenu, à une température de 250°C, par injection de chlorure d'hydrogène.
L'utilisation d'une filière de diamètre 2,4 mm et du chlorure d'hydrogène comme agent chlorant rend non conforme à l'invention le catalyseur ainsi préparé.

EXEMPLE 5 Catalyseur E (conforme à l'invention)

L'alumine gamma est mise en forme par extrusion au travers d'une filière de diamètre 1,2 mm. Après calcination sous air préalable, on dépose sur ladite alumine mise en forme 0,3 % de platine par échange ionique avec de l'acide hexachloroplatinique en présence d'HCl comme agent compétiteur. Le solide ainsi préparé est calciné sous air puis réduit sous hydrogène à 600°C.
On procède ensuite à la chloration du solide obtenu, à une température de 250°C, par injection du chloroforme.

EXEMPLE 6 Catalyseur F (conforme à l'invention)

Un mélange 90 % poids d'alumine êta et 10 % d'alumine gamma est mis en forme par extrusion au travers d'une filière de diamètre 1,2 mm. Après calcination sous air préalable, on dépose sur ladite alumine mise en forme 0,3 % de platine par échange ionique avec de l'acide hexachloroplatinique en présence d'HCl comme agent compétiteur. Le solide ainsi préparé est calciné sous air puis réduit sous hydrogène à 600°C.
On procède ensuite à la chloration du solide obtenu, à une température de 250°C, par injection de tétrachlorure de carbone.

EXEMPLE 7 Catalyseur G (conforme à l'invention)

Un mélange 90 % poids d'alumine êta et 10 % d'alumine gamma est mis en forme par extrusion au travers d'une filière de diamètre 1,2 mm. Après calcination sous air préalable, on dépose sur ladite alumine mise en forme 0,3 % de platine par échange ionique avec de l'acide hexachloroplatinique en présence d'HCl comme agent compétiteur. Le solide ainsi préparé est calciné sous air puis réduit sous hydrogène à 600°C.

On procède ensuite à la chloration du solide obtenu, à une température de 250°C, par injection de chloroforme.

EXEMPLE 8 Comparaison de la chloration des catalyseurs A, B , C, D, E, F, G

La teneur en chlore en pourcentage poids pour les catalyseurs A, B, C, D, E, F, G est suivie en fonction de la durée de chloration par fluorescence X.

Seuls les catalyseur A,E,F,G sont conformes à l'invention.

Tableau 1

| Durée de chloration | 1 heure | 3 heures | 6 heures | 9 heures |
|---|---|---|---|---|
| Teneur Cl catalyseur A | 5,4 % | 5,9% | 6,2% | 6,4% |
| Teneur Cl catalyseur B | 2,9% | 3,5% | 3,7% | 3,8% |
| Teneur Cl catalyseur C | 2,7% | 3,7% | 4,3% | 4,4% |
| Teneur Cl catalyseur D | 2,8% | 3,2% | 3,5% | 3,7% |
| Teneur Cl catalyseur E | 5,0% | 5,7% | 6,1% | 6,2% |
| Teneur Cl catalyseur F | 8,5% | 8,7% | 9,1% | 9,8% |
| Teneur Cl catalyseur G | 7,9% | 8,4% | 8,9% | 9,2% |

Pour les deux catalyseurs chlorés par le tétrachlorure de carbone (A et C) il apparaît nettement dans le tableau 1 que le catalyseur A atteint une teneur en chlore plus forte que le catalyseur C, et ceci beaucoup plus rapidement.

Pour les deux catalyseurs chlorés par le chlorure d'hydrogène (B et D), on ne voit pratiquement pas d'écart entre les teneurs en chlore des catalyseurs B et D. Toutefois la chloration par HCl donne une teneur en chlore maximale inférieure à celle obtenue par chloration avec $CCl_4$.

Dans le cas de la chloration par HCl, le diamètre des extrudés semble peu jouer sur la teneur en chlore maximale et sur la vitesse de chloration. Par contre dans le cas de la chloration par $CCl_4$, le diamètre des extrudés joue considérablement sur la teneur en chlore maximale et sur la vitesse de chloration.

Les catalyseurs E et G chlorés par le chloroforme ont des teneurs en chlore obtenues proches de celles obtenues par chloration avec le tétrachlorure de carbone (catalyseurs A et F).

Les catalyseurs F et G contenant de l'alumine êta et de l'alumine gamma ont des teneurs en chlore obtenues sensiblement égales.

Finalement, l'utilisation selon les exemples 1 à 8 d'un support extrudé dans une filière à 1,2 mm permet de réduire les temps de chloration tout en maintenant une teneur en chlore maximale sur le catalyseur (qui atteint rapidement un minimum de 4,5 % poids de chlore).

EXEMPLE 9 Test d'isomérisation des normales paraffines $C_5$-$C_6$

Les catalyseurs A et C préparés précédemment sont chacun testés en isomérisation d'une charge formée d'environ 60 % de normales paraffines $C_5$ et de 40 % de normales paraffines $C_6$, ladite charge contenant 100 ppm de $CCl_4$ exprimé en poids de chlore pour maintenir la teneur en chlore du catalyseur utilisé.

Les conditions opératoires sont les suivantes :

- Température        : 150°C

- Pression        : 2 MPa

- v.v.h        :2h$^{-1}$

- $H_2$/HC (dans l'effluent)        : 0,07

Les performances obtenues après 24 heures de fonctionnement sont reportées dans le tableau 2.

Les rapports $iC_x/(i + n)\ C_x$ où x = 5,6, où $iC_x$ représente la quantité d'isoparaffines à x atomes de carbone dans l'effluent et $(i + n)C_x$ représente la quantité d'isoparaffines et de normales paraffines à x atomes de carbone dans l'effluent.

Les approches à l'équilibre sur les différents isomères sont définies comme suit :

$$AEQi_x = \frac{iC_x/(i + n)\ C_x \text{ dans l'effluent}}{iC_x/(i + n)\ C_x \text{ à l'équilibre}}$$

avec $i_x$ = isoparaffine à x atomes de carbone (x = 5 ou 6)

Tableau 2

|  | Catalyseur A (selon l'invention) | Catalyseur C (comparatif) |
|---|---|---|
| Teneur en Cl (%poids) | 5,9 | 3,7 |
| $iC_5/(i + n)C_5$ | 0,78 | 0,59 |
| $iC_6/(i + n)C_6$ | 0,89 | 0,73 |
| $AEQi_5$ (%) | 94,0 | 71,0 |
| AEQ 2,2 diméthylbutane (%) | 88,0 | 60,0 |
| Craquage (% poids) (réaction secondaire) | 2,0 | 1,2 |

Le catalyseur A conforme à l'invention avec une teneur en chlore supérieure à 4,5% donne de meilleures performances que le catalyseur C dont la teneur en chlore est plus faible.

**Revendications**

1. Catalyseur contenant au moins un métal du groupe VIII, un support mis en forme comprenant de l'alumine gamma et de plus petite dimension moyenne comprise entre 0,8 et 2 mm et du chlore à une teneur comprise entre 4,5 et 15 % en poids déposé à partir de tétrachlorure de carbone ou de chloroforme.

2. Catalyseur selon la revendication 1 tel que le support comprend en outre de l'alumine êta.

3. Catalyseur selon l'une des revendications 1 ou 2 tel que le support est formé essentiellement d'extrudés.

4. Catalyseur selon l'une des revendications 1 ou 2 tel que le support est formé essentiellement de billes de diamètre moyen compris entre 0,8 et 2 mm.

5. Catalyseur selon l'une des revendications 1 à 4 tel que l'alumine gamma a une surface spécifique comprise entre 150 et 300 $m^2$/g et un volume poreux compris entre 0,4 et 0,8 $cm^3$/g

6. Catalyseur selon l'une des revendications 1 à 5 tel que l'alumina êta, si elle est présente dans le support dudit catalyseur, a une surface spécifique comprise entre 400 et 600 $m^2$/g et un volume poreux compris entre 0,3 et 0,5 $cm^3$/g.

7. Catalyseur selon l'une des revendications 1 à 6 tel que le métal du groupe VIII est choisi dans le groupe formé par le platine, le palladium et le nickel.

8. Catalyseur selon l'une des revendications 1 à 7 dans lequel la teneur en chlore est comprise entre 5 et 12 % poids.

9. Utilisation du catalyseur selon l'une des revendications 1 à 8, dans un procédé d'isomérisation d'une charge comprenant en majeure partie des normales paraffines comprenant de 4 à 6 atomes de carbone par molécule.

10. Utilisation selon la revendication 9 dans laquelle la charge comprend en majeure partie des normales paraffines comprenant de 5 à 6 atomes de carbone par molécules.

**Claims**

1. A catalyst containing at least one halogen, at least one metal from group VIII, a formed support comprising gamma alumina and of smallest average dimension in the range 0.8 mm to 2 mm and chlorine with a content is in the range 4.5% to 15% by weight, deposited from carbon tetracloride or chloroform.

2. A catalyst according to claim 1, wherein the support also comprises eta alumina.

3. A catalyst according to claim 1 or 2, wherein the support is essentially formed of extrudates.

4. A catalyst according to claim 1 or 2, wherein the support is essentially formed of spherules with an average diameter in the range 0.8 mm to 2 mm.

5. A catalyst according to anyone of claim 1 to 4, wherein the gamma alumina has a specific surface area in the range 150 m$^2$/g to 300 m$^2$/g and a pore volume in the range 0.4 cm$^3$/g to 0.8 cm$^3$/g.

6. A catalyst according to anyone of cliams 1 to 5 wherein eta alumina, if present in the support of said catalyst, has a specific surface area in the range 400 m$^2$/g to 600 m$^2$/g and a pore volume in the range 0.3 cm$^3$/g to 0.5 cm$^3$/g.

7. A catalyst according to anyone of claim 1 to 6 wherein the metal of group VIII is selected from the group formed by platinum, palladium and nickel.

8. A catalyst according to anyone of claims 1 to 7 wherein the chlorine content is in the range 5 % to 12 % weight.

9. The use of a catalyst according to anyone of claim 1 to 8, in an isomerisation process for a feed mainly comprising normal paraffins containing 4 to 6 carbon atoms per molecule.

10. The use according to claim 9 wherein the feedstock comprises mainly normal paraffins containing 5 to 6 carbon atoms per molecule.

**Patentansprüche**

1. Katalysator, wenigstens ein Metall der Gruppe VIII, einen geformten Träger, der Gamma-Aluminiumoxid umfasst und dessen kleinste mittlere Abmessung zwischen 0,8 und 2 mm liegt sowie Chlor bei einem Gehalt zwischen 4,5 und 15 Gew.-% enthaltend, das aus Tetrachlorkohlenstoff oder Chloroform abgeschieden ist.

2. Katalysator nach Anspruch 1, der Art, dass der Träger im Übrigen Eta-Aluminiumoxid umfasst.

3. Katalysator nach einem der Ansprüche 1 oder 2, der Art, dass der Träger im Wesentlichen aus Extrudaten geformt ist.

4. Katalysator nach einem der Ansprüche 1 oder 2, der Art, dass der Träger im Wesentlichen aus Kugeln eines mittleren Durchmessers zwischen 0,8 und 2 mm geformt ist.

5. Katalysator nach einem der Ansprüche 1 bis 4, der Art, dass das Gamma-Aluminiumoxid eine spezifische Oberfläche zwischen 150 und 300 m$^2$/g und ein Porenvolumen zwischen 0,4 und 0,8 cm$^3$/g hat.

6. Katalysator nach einem der Ansprüche 1 bis 5, der Art, dass das Eta-Aluminiumoxid, wenn es im Träger dieses Katalysators vorhanden ist, eine spezifische Oberfläche zwischen 400 und 600 m$^2$/g und ein Porenvolumen zwischen 0,3 und 0,5 cm$^3$/g hat.

7. Katalysator nach einem der Ansprüche 1 bis 6, der Art, dass das Metall der Gruppe VIII gewählt ist aus der durch Platin, Palladium und Nickel gebildeten Gruppe.

8. Katalysator nach einem der Ansprüche 1 bis 7, bei dem der Gehalt an Chlor zwischen 5 und 12 Gew.-% liegt.

9. Verwendung des Katalysators nach einem der Ansprüche 1 bis 8 bei einem Verfahren zur Isomerierung einer Charge, die zum überwiegenden Teil Normalparaffine umfasst, die 4 bis 6 Kohlenstoffatome pro Molekül aufweisen.

10. Verwendung nach Anspruch 9, bei dem die Charge zum überwiegenden Teil Normalparaffine umfasst, die 5 bis 6 Kohlenstoffatome pro Molekül aufweisen.